Europäisches Patentamt

European Patent Office

Office européen des brevets

(11) Publication number: **0 448 513 A2**

# (12) EUROPEAN PATENT APPLICATION

(21) Application number: **91810163.5**

(22) Date of filing: **12.03.91**

(51) Int. Cl.⁵: **C12N 15/58, C12N 9/02, C07K 15/00, C07K 1/00**

The applicant has filed a statement in accordance with Rule 28 (4) EPC (issue of a sample only to an expert). Accession number(s) of the deposit(s):

(30) Priority: **21.03.90 GB 9006354**
**10.08.90 JP 210535/90**
**30.11.90 GB 9026082**

(43) Date of publication of application:
**25.09.91 Bulletin 91/39**

(84) Designated Contracting States:
**AT BE CH DE DK ES FR GB GR IT LI LU NL SE**

(71) Applicant: **Japat Ltd**
**Klybeckstrasse 141**
**CH-4057 Basel (CH)**

(72) Inventor: **Suzuki, Kenji**
**1-1-45-305, Miyayama-cho**
**Toyonaka-shi, Osaka (JP)**
Inventor: **Shimoi, Hiroko**
**16-17, Tsukimigaoka, Nigawa**
**Takarazuka-shi, Hyogo (JP)**
Inventor: **Iwasaki, Yasuno**
**1-12-48, Hobai**
**Takarazuka-shi, Hyogo (JP)**
Inventor: **Nishikawa, Yoshiki**
**2-48-16, Seiwa-dai, Kita-ku**
**Kobe-shi, Hyogo (JP)**

(74) Representative: **Schluep, Hans-Peter et al**
**c/o CIBA GEIGY AG Patentabteilung Postfach**
**CH-4002 Basel (CH)**

(54) **Process for production of peptidylglycine alpha-hydroxylating monooxygenase and use thereof.**

(57)     A process for the production of peptidylglycine α-hydroxylating monooxygenase, comprising the steps of:
culturing insect cells transfected with a recombinant baculovirus to which a DNA coding for the peptidylglycine α-hydroxylaring monooxygenase has been incorporated to produce the enzyme, and recovering the enzyme from the culture; and a process for the production of a C-terminal α-hydroxyglycyl peptide, comprising converting a C-terminal glycine-extended peptide to a C-terminal α-hydroxyglycyl peptide using the enzyme prepared by the above process.

EP 0 448 513 A2

# Fig. 2

pAcYM1/AE-I

AUTOGRAPHA CALOFORNICA
NUCLEAR POLYHEDROSIS VIRUS

WILD-TYPE AcNPV DNA

Co-TRANSFECTION

INSECT CELLS

HOMOLOGOUS RECOMBINATION

4-5 DAYS INCUBATION

HARVEST SUPERNATANT

PLAQUE ASSAY

POLYHEDRIN-NEGATIVE
CLEAR-PLAQUE

WILD-TYPE
WHITE PLAQUE

CLEAR-PLAQUE ISOLATION

RECOMBINANT VIRUS DNA

POLYHEDRIN
PROMOTOR

AMPLIFICATION

# PROCESS FOR PRODUCTION OF PEPTIDYLGLYCINE α-HYDROXYLATING MONOOXYGENASE AND USE THEREOF

## Field of the Invention

The present invention relates to a process for the production of peptidylglycine α-hydroxylating monooxygenase (PAM), to the use thereof for preparing a C-terminal α-hydroxyglycyl peptide from a corresponding glycine-extended peptide and said α-hydroxyglycyl peptide per se.

## Description of the Prior Art

Designated peptide C-terminal α-amidating enzymes are a group of enzymes which catalyze the conversion of a glycine-extended peptide represented by the formula $R-NH-CH_2-COOH$, wherein R represents a peptide and $-NH-CH_2-COOH$ represents a glycine residue present at the C-terminus of the peptide, to an α-amidated peptide represented by the formula $R-NH_2$ wherein the amino group amidates the α-carboxyl group of the C-terminal amino acid of the peptide.

It is well known that many biologically active peptides have a C-terminal α-amidated structure, and in many cases this structure is essential to their biological activity, but there are difficulties in the direct preparation of a C-terminal α-amidated peptide by chemical synthesis and gene recombination techniques. To remove this difficulty, many attempts have been made to use a peptidylglycine α-amidating enzyme to prepare a C-terminal α-amidated peptide. For example, EP 0308 067 describes an α-amidating enzyme composition of MTC tumors origin, and the use thereof

Mizuno K., et al., Biochem. Biophys. Res. Comm. Vol. 148, No. 2, pp 546-552, 1987 describes an α-amidating enzyme AE-I of Xenopus laevis skin origin; and Ohsuye K., et al., Biochem. Biophys. Res. Comm. Vol. 150. No. 3, pp. 1275-1281, 1988 describes another type of α-amidating enzyme AE-II of Xenopus laevis skin origin. EP 0299790 discloses a cloning of cDNAs coding for the enzymes of the Xenopus laevis origin in plasmids pXA457 and pXA799, respectively. Nevertheless, the expression of the cDNA in E. coli cells provides a product with only marginal activity, probably due to incorrect folding of the expressed enzyme or to other reasons, one of which could be that the product has no real α-amidating activity.

## Object of the invention

It was now surprisinbly found that X. laevis AE-I is not a real α-amidating but an α-hydroxylating enzyme and that the above-mentioned conversion of $R-NH-CH_2-COOH$ to $R-NH_2$ occurs in two steps. The first step is catalyzed by a peptidylglycine α-hydroxylating monooxygenase and leads to an intermediate product $R-NH-CHOH-COOH$. It is to be supposed that also some other of the above-mentioned α-amidating enzymes are not real amidating enzymes but catalyse only the first step of the reaction and, therefore, are peptidylglycine α-hydroxylating monooxygenases.

There is a need for a process for the production of peptidylglycine α-hydroxylating monooxygenase having a high potency. As a means for the production of a biologically active peptide or an enzyme, USP No. 4,745,051 discloses the use of a baculovirus-insect cell expression system.

It is an object of the present invention to provide a method for the production of a peptidylglycine α-hydroxylating monooxygenase having a high potency and a method for the production of α-hydroxyglycyl peptides using said α-hydroxylating monooxygenase.

## Description of the invention

The present invention provides a process for the production of a peptidylglycine α-hydroxylating monooxygenase having a high potency by a gene recombination method using a baculovirus-insect cell expression system.

More specifically, the present invention provides a process for the production of a peptidylglycine α-hydroxylating monooxygenase, said method comprising the steps of: culturing insect cells transfected with a recombinant baculovirus to which a DNA coding for the peptidylglycine α-hydroxylating monooxygenase has been incorporated to produce the enzyme; and optionally recovering the enzyme from the culture.

The present invention moreover provides a process for the production of a C-terminal α-hydroxglycyl peptide, comprising converting a C-terminal glycine-extended peptide of the formula (I)

$$R-NH-CH_2-COOH \quad (I)$$

wherein R represents a peptide and -NH-CH$_2$-COOH represents a glycine residue present at the C-terminal of the peptide R, to a C-terminal α-hydroxyglycyl peptide of the formula (II)

$$R-NH-CHOH-COOH \quad (II)$$

using the enzyme prepared by a process according to the present invention.

The peptidylglycine α-hydroxylating monooxygenases of the present invention include any enzymes having the above-defined C-terminal α-hydroxylating activity, and derived from various origins. Preferably the present peptidylglycine α-hydroxylating enzymes are those disclosed in the above-mentioned literature, such as AE-I and AE-II of Xenopus laevis skin origin, as well as fragments thereof having the desired enzyme activity. Most preferably, the present enzyme is AE-I.

The DNA coding for the present enzyme may be prepared according to a conventional procedure. For example, total RNA is extracted from a material in which an α-hydroxyglycyl or α-amidated peptide is natively expressed such as the skin of Xenopus laevis, bovine intermediate pituitary gland, rat atrium, or the like according to a conventional procedure. Next, poly (A)+RNA is isolated by oligo dT-cellulose column chromatobraphy according to a conventional procedure such as that of Chirgwin, J. M. et al., Biochemistry 18, 5294-5299 (1977). Next, a cDNA library is constructed according to a conventional procedure, for example in λ phage, or according to the method of Okayama and Berg, Molec. Cell Biol. 2, 161 - 170, 1982; and the cDNA library is screened by a hybridization method using a synthetic oligonucleodite probe designed according to a published sequence of cDNA encoding peptidylglycine α-hydroxylating monooxygenase, derived from, for example, bovine, frog or rat (see Mizuno, K. et al. (1987), Biochem. Biophys. Res. Comm. 148, 546-552; Eipper, B.A. et al. (1987), Molec. Endocrinology 1, No. 11, 777-790; Ohsuye, K. et al. (1988), Biochem. Biophs. Res. Comm. 150, 1275-1281; Stoffers, D.A. et al. (1989), Proc. Natl. Acad. Sci. USA 86, 735-739). In a preferred embodiment of the present invention, a cDNA is cloned using an oligonucleotide probe synthesized according to the DNA sequence of AE-I described in EP 0299790.

According to the present invention, a DNA coding for the present enzyme, such as cDNA, is inserted into a baculovirus transfer vector to construct a recombinant baculovirus transfer vector, and the recombinant baculovirus transfer vector is then co-transfected with a baculovirus DNA to insect cells to carry out a homologous recombination. The baculovirus transfer vector is usually a plasmid containing a segment of baculovirus DNA, which segment comprises a gene not essential for the replication of baculovirus. The gene not essential for the replication of baculovirus is, for example, a polyhedrin gene comprising a polyhedrin structure gene and a promoter thereof. Such baculovirus transfer vectors are, for example, pAcYM1 (Matsuura, Y., et al., J. Gen. Virol. (1987) 68, 1233- 1250); pAc311, pAc360, pAc373 and pAc380 (USP 4,745,051 ), and the like.

The baculoviruses used in the present invention are, for example, Autographa californica nuclear polyhedrosis virus (AcMNPV), Trichoplusia ni MNPV, Rachiplusia ou MNPV, Galleria mellonella MNPV, and the like.

A kit comprising a combination of an Autographa californica nuclear polyhedrosis virus and baculovirus transfer vectors pAc700, pAc701, pAc702, pVL1392 and pVL1393 are commercially available from Invitrogen.

The insect cells used in the present invention are established insect cell lines, for example, Spodoptera frugiperda, such as Sf9 (ATTC CRL1711), Sf21, Mamestra brassicae and the like.

Bombyx mori cell system using Bombyx mori nuclear polyhedrosis virus (BmNPV) is also available for the present invention.

The homologous recombination is carried out in accordance with a conventional procedure, as described in Example 3. According to the present invention, the homologous recombination occurs at a rate as high as several percents, and a successful recombinant virus can be selected as a polyhedrin-negative clear plaque from among wild-type white plaques. Accordingly, a desired recombinant virus can be easily and reproducibly selected.

The transfected insect cells are cultured in accordance with a conventional procedure. Namely, the transfected insect cells may be cultured in any tissue culture medium in which insect cells can grow, such as Grace's or TC100 medium supplemented with mammalian serum, serum-free medium EX-CELL400, or the like, at a temperature of 20°C to 30°C, preferably 27°C to 28°C, for example 27°C, for 2 to 10 days, preferably 3 to 5 days. According to the present invention, more than 90 % of an expressed enzyme is secreted into the culture supernatant, and therefore, the desired enzyme can be recovered from the culture supernatant according to a conventional procedure, such as centrifugation, a salting out technique, or various chromatographic procedures, or a combination thereof.

When the purified enzyme thus prepared is reacted upon a glycine extended peptide of the formula (I), e.g. upon the model peptide Ala-Ile-Gly-Val-Gly-Ala-Pro-Gly being identical with the 7 C-terminal amino acid residues of human calcitonin extended with a C-terminal glycine residue, or upon C-terminally glycine extended precursors of calcitonin such as human calcitonin, salmon calcitonin, chicken calcitonin, eel calcitonin, rat calcitonin, porcine calcitonin, ovine calcitonin, bovine calcitonin, or upon human growth hormone-releasing factor (GHRF), human calcitonin gene-related peptide (CGRP), luteinizing hormone-releasing hormone (LHRH), and the like, the corresponding α-hydroxyglycyl peptides of the formula (II) are produced.

The production of such an α-hydroxyglycyl peptide is carried out using a peptidylglycine α-hydroxylating monooxygenase-containing preparation, starting from a corresponding glycine-extended peptide in an aqueous medium such as a buffer, for example, a phosphate buffer, acetate buffer, MES-Na buffer, TES-Na buffer, Tris-HCl buffer, or the like, at a temperature of 16°C to 37 °C, preferably about 30°C. An amount of the enzyme used is for example $1 \times 10^2$ to $1 \times 10^6$ units/mg of substrate protein and preferably $1 \times 10^3$ to $5 \times 10^5$ units/mg of substrate protein. The peptidylglycine α-hydroxylating monooxygenase-containing preparation may be any preparation containing said enzyme, such as a culture supernatant, a concentrated supernatant, an enriched or partially purified enzyme preparation, a purified enzyme, or the like. Such enzyme preparations can be lyophilized for storage.

The α-hydroxyglycyl peptides R-NH-CHOH-COOH produced by the above process are any α-hydroxglycyl peptides including the precursors of calcitonins, such as human calcitonin, salmon calcitonin, chicken calcitonin, eel calcitonin, rat calcitonin, porcine calcitonin, ovine calcitonin, bovine calcitonin, or of human growth hormone-releasing factor (GHRF), human calcitonin gene-related peptide (CGRP), luteinizing hormone-releasing hormone (LHRH), and the like. Preferred are the precursors for calcitonin, most preferentially human calcitonin. Accordingly, the present invention also concerns the C-terminal α-hydroxyglycyl peptides of the formula R-NH-CHOH-COOH mentioned above, the production thereof by means of a peptidylglycine α-hydroxylating monooxygenase and the use thereof for producing the corresponding α-amidated peptides of the formula R-NH$_2$ wherein the amino group amidates the α-carboxyl group of the C-terminal amino acid of the peptide.

R-NH-CHOH-COOH is surprisingly stable at physiological conditions and can be converted to R-NH$_2$ by the addition of a base, e.g. an alkali hydroxyde, in particular NaOH, at a basic pH value, particularly at about pH 9 to about pH 12, preferentially about pH 10 to about pH 11, e.g. by incubation for a few minutes at pH 11 or for 30 minutes at pH 10.

## Description of the drawings

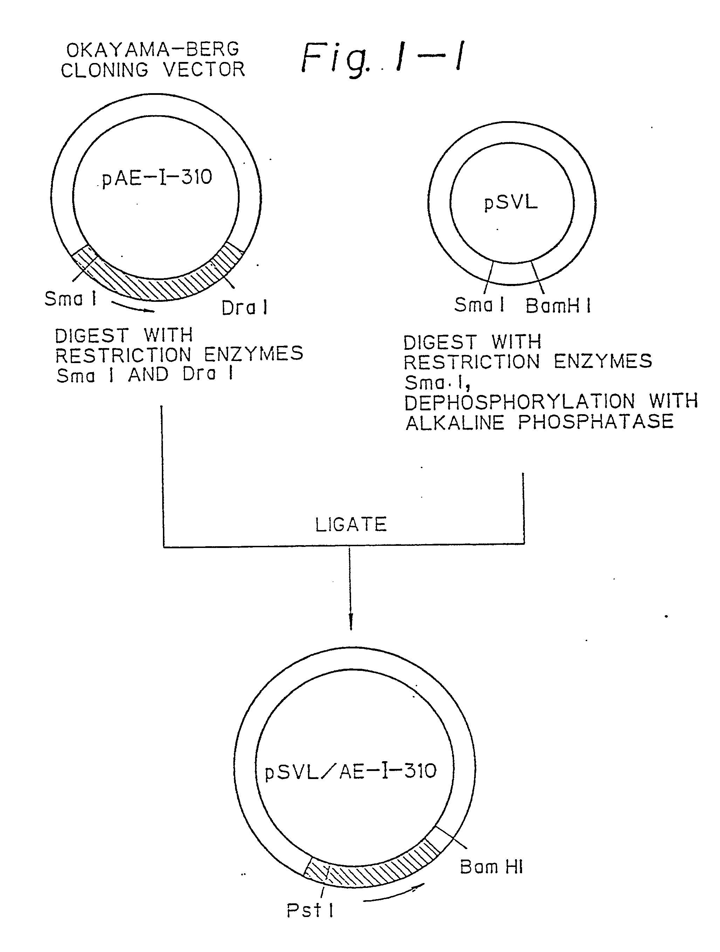

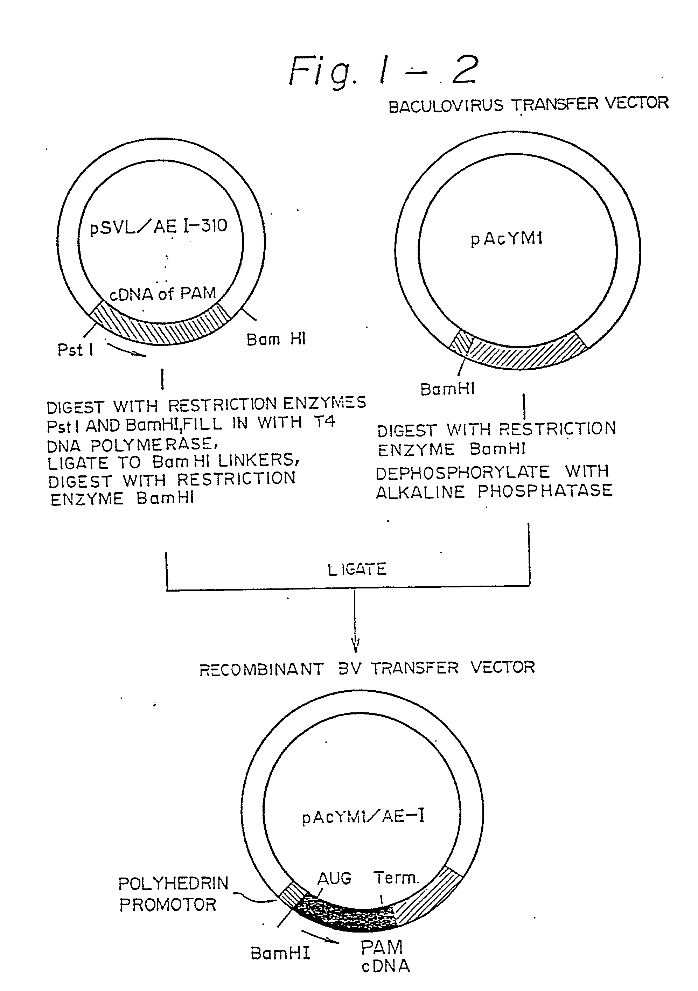

Fig. 1-1 represents a process for the construction of the intermediate plasmid pSVL/AE-I-310;

Fig. 1-2 represents a process for the construction of the recombinant baculovirus transfer vector pAcYM1/AE-I;

Fig. 2 represents a process for the construction of a recombinant baculovirus;

Fig. 3 represents the production of the peptidylglycine α-hydroxylating monooxygenase by insect cells transfected with the recombinant baculovirus in a serum-free medium; and

Fig. 4 represents the time-dependent conversion of glycine-extended human calcitonin (hCT-Gly) to human calcitonin (hCT), using an enriched insect cell culture medium.

## Examples

The following examples serve to illustrate the present invention but should not be construed as a limitation thereof.

## Example 1: Cloning of cDNA coding for peptidyl-glycine α-hydroxylating monooxygenase AE-I from Xenopus laevis skin

Total RNA is extracted from the skins of Xenopus leavis and poly(A)$^+$RNA is isolated by oligo(dT)-cellulose column chromatography according to a procedure of Chirgwin, J.M. et al., Biochemistry 18, 5294-5299 ( 1977 ). A cDNA library is constructed by the method of Okayama and Berg, Molec. Cell. Biol. 2, 161-170, 1982, using 10 μg of poly(A)$^+$RNA and 5 μg of the vector-primer DNA. The 29 bp synthetic oligonucleotide (CAT-CACATGCTTCTATTTGGATGCAATAT) which corresponds to [103]His-[112]Ile in AE-I [Mizuno K., et al., Biochem. Biophys. Res. Comm. 148, 546-552 (1987)] is used as a hybridization probe, according to the published sequence of Xenopus laevis hydroxylating enzyme, AE-I. One of the positive clones, pAE-I-310, is found to contain cDNA of the Xenopus laevis hydroxylating enzyme, AE-I, by restriction mapping and nucleotide sequence analysis. Nucleotide sequence of the cDNA flanking SmaI and DraI ends is shown in the Sequence Listing/SEQ ID No. 1.

## Example 2: Construction of the recombinant transfer vector pAcYM1/AE-I (Fig. 1)

The starting transfer vector pAcYM1 is a plasmid containing a polyhedrin gene of baculovirus, described in detail in Matsuura Y., et al., J. Gen. Virol. (1987) 68, 1233-1250.

To construct a transfer vector for an insect cell expression system, the cDNA clone pAE-I-310 is double digested with restriction enzymes SmaI and DraI.

The SmaI/DraI fragment is then integrated into the site of SmaI in pSVL vector (Pharmacia). The plasmid of the desired orientation is named pSVL/AE-I-310. Then the plasmid is double digested with restriction enzymes PstI and BamHI. A resulting 1.3 Kbp fragment of the clone contained a coding region for the hydroxylating enzyme AE-I. This fragment is blunted with T4 DNA polymerase, followed by ligation with a BamHI linker and digestion with the restriction enzyme BamHI. pAcYM1/AE-I is prepared by inserting the modified fragment into the baculovirus transfer vector pAcYM1 which is previously treated with the restriction enzyme BamHI and dephosphorylated.

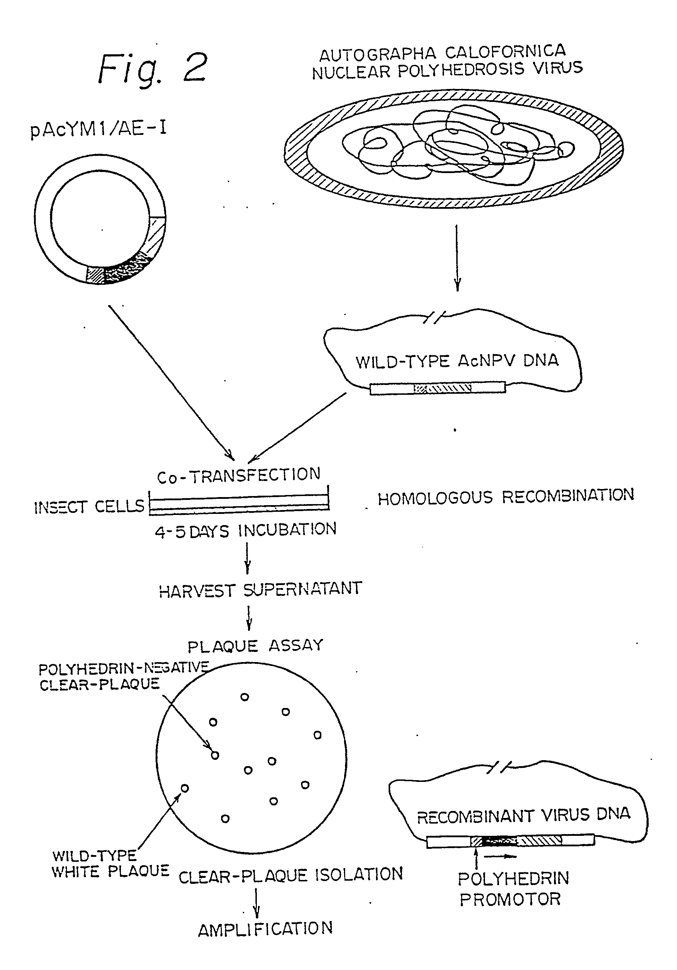

Example 3: Construction of recombinant baculovirus (Fig. 2)

The transfer plasmid containing an AE-I cDNA is recombined into the wild type Autographa californica nuclear polyhedrosis virus (AcMNPV) genome by cell-mediated homologous recombination. The wild type AcMNPV is commercially available from Invitrogen. Then 1 μg of the viral DNA is mixed with 25- 100 μg of the plasmid pAcYM1/AE-I DNA in 950 μl of HEPES buffer (20 mM HEPES (pH 7.0) / 1 mM $Na_2HPO_4$ / 5 mM KCL / 140 mM NaCl / 10 mM glucose), and after precipitation with 50 μl of 2.5 M $CaCl_2$, DNA particles are placed onto $2 \times 10^6$ Sf9 cells and the culture is incubated for 1 hour at room temperature. Then the supernatant of the culture is replaced by 2 ml of Grace's medium (GIBCO) containing 10 % fetal bovine serum (GIBCO). After 4 days of incubation at 27°C, the supernatant is harvested and diluted 10 to 1000 fold, followed by the plaque assay. The plaques that will form from a recombinant baculovirus having an insertion of foreign DNA into their genome may be distinguished from non-recombinant viral plaques by visual screening. Two polyhedrin-negative plaques are picked by visual screening, and the further purification and amplification of the recombinant viruses are performed according to a standard procedure (A Manual of Methods for Baculovirus Vectors and Insect Cell Culture Procedures, M.D. Summers, Texas Agricultural Experiment Station Bulletin No. 1555).

Example 4: Production of enzyme

First, $5 \times 10^6$ Sf9 (ATTC CRL1711) cells are mixed with the isolated recombinant viruses at a MOI (multiplicity of infection) of 10 and supplemented with 8 ml of EX-CELL 400 (J R Scientific), a serum-free medium. The infected insect cells are cultured in the EX-CELL 400 medium at 27°C for 7 days.

The insect cell culture medium prepared as described above is centrifuged and the supernatant is concentrated 40-fold by using an Amicon concentrator, and the concentrated sample is applied onto a Sephadex G-25 gel filtration column, PD-10 (Pharmacia), to remove low molecular weight materials. The enzyme activity eluted from the column is stable for at least 2 months at -20°C.

The enzyme activity of peptidylglycine α-hydroxylating monooxygenase is measured according to Jones, B.N. et al. (Anal. Biochem. 168, 272-279, 1988), with a slight modification. A standard assay condition is performed at pH 6.4 in 200 mM TES-Na / 2 mM ascorbic acid / 50 mM KJ / 1 μ $CuSO_4$ / 2 mM N-ethylmaleimide / 20 μM N-dansyl-Tyr-Phe-Gly / 100 μg/ml catalase, and the reaction is started by the addition of an enzyme at 30°C and stopped after 15-30 min by the addition of EDTA to a final concentration of 50 mM, followed by an addition of NaOH to a final concentration of 0.2 N and neutralization with HCl. The sample is analyzed on a Hi-pore (4.6 x 40 mm) column (Bio-Rad) using an isocratic condition of 62.5 mM $CH_3COONa$ / 33 % acetonitrile, pH 7.0. One unit of the enzyme activity is defined as the amount of enzyme to produce 1 pmole of the product per minute.

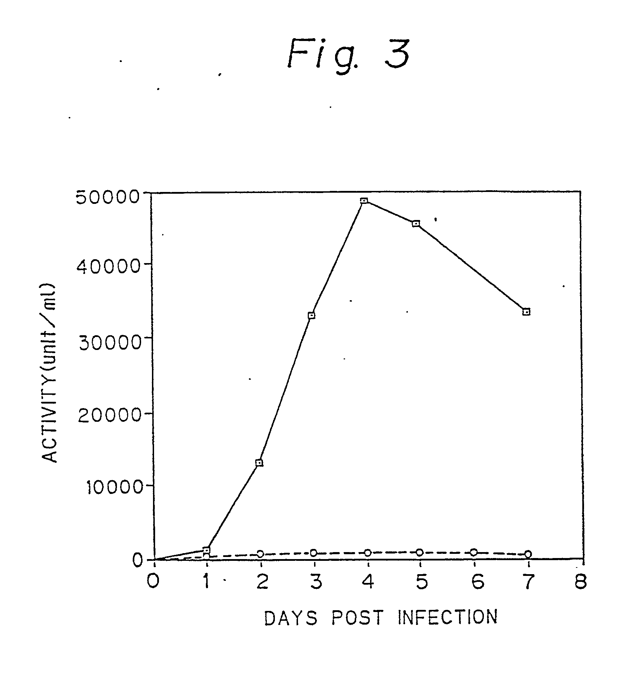

The result of the culture is shown in Fig. 3.

A solid line indicates activity measured according to the method described above. A dotted line indicates activity measured without alkaline treatment and neutralization. Note, the α-hydroxyglycine extended peptide can be converted to the corresponding αamidated peptide by alkaline treatment, for example for a few minutes at pH 11 or for 30 minutes at pH 10.

To purify the enzyme, the culture media (500 ml) prepared as above is treated as follows. After removing the cells by low-speed centrifugation, the medium is dialyzed against 10 mM Tris-Cl / 0.1 % Lubrol, pH 7.5 (Buffer A), and applied to a 2.6 x 22 cm column of DEAE-Cellulose (DE52) equilibrated with the Buffer A. The hydroxylating enzyme activity is eluted with a linear salt gradient of 0-1.0 M NaCl in the Buffer A, and fractions containing the hydroxylating enzyme activity are collected, dialyzed against 50 mM MES-Na, pH 5.7 and applied to a Mono S HR5/5 column (Pharmacia) equilibrated with 50 mM MES-Na, Ph 5.7. The hydroxylating enzyme is eluted with a linear salt gradient of 0-500 mM NaCl in the same buffer. Fractions with the high activity are pooled and dialyzed against the Buffer A, followed by loading to a DEAE GLASS column (Nacalai) equilibrated with the Buffer A. The hydroxylating enzyme is eluted with a linear gradient of 0-500 mM of NaCl, in the Buffer A.

The result of the purification is shown in Table 1.

## Table 1

| Purification step | Protein (mg) | Activity (unit) x $10^6$ | Specific activity (u/mg) x $10^4$ | Recovery (%) | Purification (fold) |
|---|---|---|---|---|---|
| Medium | 504 | 10.5 | 2.08 | 100 | 1.0 |
| DE52 | 40.6 | 5.94 | 14.6 | 57 | 7.0 |
| Mono S | 1.08 | 1.93 | 179 | 18 | 86 |
| DEAE GLASS | 0.70 | 1.50 | 214 | 14 | 103 |

The purified protein is verified to be homogeneous on denaturing SDS-polyacrylamide gel electrophoresis, revealing its molecular weight of 41 kDa. An apparent molecular weight determined from gel filtration is 43 kDa.

Note, for the hydroxylation of peptides it is unnecessary to purifiy peptidyl glycine α-hydroxylating monooxygenase to 100 % homogenity and impurities do not disturb the hydroxylation of glycine-extended peptides.

### Example 5: Preparation of a C-terminal α-hydroxyglycine-extended human calcitonin

Twenty six mg of C-terminal glycine extended human calcitonin is added in 0.2 M MES-Na containing 2 mM L-ascorbate, 0.1 mg/ml catalase and 10 mM KI. Then 0.6 mg of purified peptidyl glycine α-hydroxylating monooxygenase is added to the solution. After incubation at 30 °C for 5 h, the reaction solution is treated with a Sep-pak cartridge (Waters), and lyophilized. The lyophilized sample is dissolved in 90% acetic acid and applied into a Bio-Rad Hi Pore RP 304 (10 x 250 mm) column. The elution is conducted with a linear acetonitrile gradient (19 - 23%) in 10 mM ammonium formate (pH 4.0). Fractions containing the product peptide are lyophilized. The analytical HPLC done using a Bio-Rad Hi Pore RP 304 (4.6 x 250 mm) column with a linear acetonotrile gradient (8 - 50%) in ammonium formate (pH 4.0) indicated that the obtained product is pure.

Analysis of the product: Thirty μg of the product obtained is subjected to Edman degradation with a gasphase protein sequencer equipped with a PTH analyzer. The amino acid structure obtained corresponds to that of the 32 amino acid chain of human calcitonin.

Five mg of the product dissolved in water (90% $H_2O$ and 10% $^2H_2O$, pH 3.2) is analyzed with $^1H$-NMR. The proton signals are assigned by DQF-COSY (Double Quantum Filtered Chemical Shift Correlated Spectroscopy). A resonance at 5.4 ppm is assigned to an α-proton in the NMR spectrum. The resonance of this α-proton shows a large downfield shift with respect to the other α-protons. This result suggests that the peptide contains an α-hydroxyglycine residue in its structure.

Twenty μ of the product are analyzed by positive ion fast atom bombardment mass spectrometry (FAB-MS). A distinct peak is obtained around mass number 3490, which is compatible with the α-hydroxyglycine extended human calcitonin. For human calcitonin, a mass number of 3420 is evaluated in FAB-MS (calculated: 3418).

### Example 6: Production of human calcitonin

To determine the time dependent conversion of glycine-extended human calcitonin, 25 mg of glycine-extended human calcitonin (hCT-Gly) is incubated with 5.25 ml of the concentrated medium obtained in Example 4 at 30°C in 15 ml of 0.2 M sodium phosphate buffer (pH 6.2) containing 5 mM L-ascorbate, 0.5 μM $CuSO_4$, 20 μg/ml catalase (Boehringer), 10 mM KI, 0.25 mM N-ethylmaleimide, 1 acetonitrile and 0.1 % Lubrol type PX. At various times during incubation, 4 μl of the aliquots are diluted 50 times with 11 % $HCOONH_4$ (pH 4) / 10 % acetonitrile, and then 25 μl of the sample is directly subjected to a HPLC column (buffer A (Mobile phase), 10 mM $HCOONH_4$ (pH 4.0) / 10 % acetonitrile; Buffer B, 10 mM $HCONH_4$ (pH 4.0) / 50 % acetonitrile. Column, Bio-Rad Hi-Pore RP 304, 4.6 mm I.D. X 250 mm).

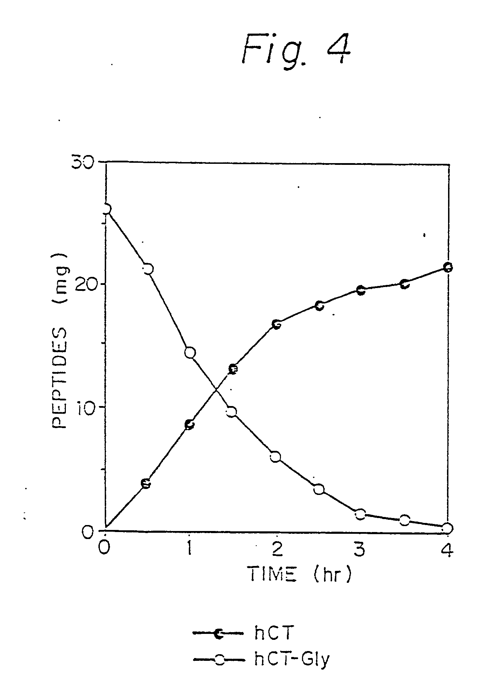

Figure 4 shows the time-dependent conversion of hCT-Gly by the expressed enzyme, wherein the open circles indicate the substrate, hCT-Gly, and the filled-in circles indicate the product, respectively.

Twenty-five mg of hCT-Gly is converted into human calcitonin (hCT) using 5 ml of a concentrated supernatant derived from 200 ml of the culture supernatant, after 4 hours incubation, the reaction is stopped by the addition of 150 μl of 0.5 M EDTA (pH 7.0) and the solution is treated with alkali as mentioned above.

Then, the reaction mixture is passed through a Seppak C-18 column (Waters), and hCT is purified on a semi-preparative reverse-phase HPLC column (YMC SH-343-5, 20 mm I:D: x 250 mm) by elution with a linear gradient of solvent A (10 mM ammonium acetate in 23 % acetonitrile) to a solvent B (the same buffer in 30 % acetonitrile) for 60 min at a flow rate of 10 ml/min. Fractions containing hCT are collected and lyophilized, and the hCT is found by chromatography to be pure. The product is also confirmed as hCT by analysis with an amino acid analyzer, a gas-phase amino acid sequencer, and a FAB-MASS spectrometer.

Deposition of microorganisms:

E. coli containing the plasmid pAcYM1 is deposited with Fermentation Research Institute Agency of Industrial Science and Technology, (FRI), 1-3, Higashi- 1-chome, Tsukuba-shi, Ibaraki-ken, Japan, as FERM BP-2796, under the Budapest Treaty, on March 7, 1990.

The plasmid pSVL/AE-I-310 is deposited with the FRI as FERM BP-2797, under the Budapest Treaty, on March 7, 1990.

8

Sequence Listing

SEQ. ID NO: 1

SEQUENCE TYPE: Nucleotide with corresponding protein

SEQUENCE LENGTH: 1391 basepairs

STRANDEDNESS: double

TOPOLOGY: Linear

MOLECULE TYPE: cDNA to mRNA

ORIGINA SOURCE

ORGANISM: Xenopus laevis

PROPERTIES:  Xenopus laevis peptidylglycine
             alpha-hydroxylating
             monooxygenase gene


```
GGGAAGAATG TGTATTTCTG GTTACCTGCG GGGCTAGCAC TGATGGAGTA      50
GGGGGGATTG ATCCAGCTTC CTAATTACCA GGATTACAAC TTGCCTTTAA     100
TTTACTCCTG CAGTAAGGCA CAGACCACAG GGTGGAC ATG GCC AGC CTC 149
                                          Met Ala Ser Leu
                                          -37      -35

AGT AGC AGC TTT CTT GTG CTC TTT CTC TTA TTT CAG AAC AGC  191
Ser Ser Ser Phe Leu Val Leu Phe Leu Leu Phe Gln Asn Ser
            -30             -25                 -20

TGC TAC TGT TTC AGG AGT CCC CTC TCT GTC TTT AAG AGG TAT  233
Cys Tyr Cys Phe Arg Ser Pro Leu Ser Val Phe Lys Arg Tyr
            -15                 -10

GAG GAA TCT ACC AGA TCA CTT TCC AAT GAC TGC TTG GGA ACC  275
Glu Glu Ser Thr Arg Ser Leu Ser Asn Asp Cys Leu Gly Thr
 -5                   1               5

ACG CGG CCC GTT ATG TCT CCA GGC TCA TCA GAT TAT ACT CTA  317
Thr Arg Pro Val Met Ser Pro Gly Ser Ser Asp Tyr Thr Leu
 10              15                  20

GAT ATC CGC ATG CCA GGA GTA ACT CCG ACA GAG TCG GAC ACA  359
Asp Ile Arg Met Pro Gly Val Thr Pro Thr Glu Ser Asp Thr
     25              30                  35

TAT TTG TGC AAG TCT TAC CGG CTG CCA GTG GAT GAT GAA GCC  401
Tyr Leu Cys Lys Ser Tyr Arg Leu Pro Val Asp Asp Glu Ala
         40              45                  50
```

```
TAT GTA GTT GAC TTC AGA CCA CAT GCC AAT ATG GAT ACT GCA    443
Tyr Val Val Asp Phe Arg Pro His Ala Asn Met Asp Thr Ala
            55              60                      65

CAT CAC ATG CTT CTA TTT GGA TGC AAT ATA CCT TCT TCC ACT    485
His His Met Leu Leu Phe Gly Cys Asn Ile Pro Ser Ser Thr
                70                  75

GAT GAT TAC TGG GAC TGT AGT GCG GGA ACT TGC ATG GAC AAA    527
Asp Asp Tyr Trp Asp Cys Ser Ala Gly Thr Cys Met Asp Lys
80                  85                  90

TCC AGT ATA ATG TAT GCC TGG GCA AAG AAT GCA CCA CCC ACC    569
Ser Ser Ile Met Tyr Ala Trp Ala Lys Asn Ala Pro Pro Thr
    95                  100                 105

AAA CTT CCA GAA GGA GTT GGC TTT CGT GTT GGA GGG AAA TCA    611
Lys Leu Pro Glu Gly Val Gly Phe Arg Val Gly Gly Lys Ser
        110                 115                 120

GGC AGT AGA TAT TTT GTG CTT CAA GTT CAC TAT GGA AAT GTG    653
Gly Ser Arg Tyr Phe Val Leu Gln Val His Tyr Gly Asn Val
            125                 130                 135

AAA GCA TTC CAG GAT AAA CAT AAA GAT TGC ACG GGG GTG ACA    695
Lys Ala Phe Gln Asp Lys His Lys Asp Cys Thr Gly Val Thr
            140                 145

GTA CGA GTA ACA CCT GAA AAA CAA CCG CAA ATT GCA GGC ATT    737
Val Arg Val Thr Pro Glu Lys Gln Pro Gln Ile Ala Gly Ile
150                 155                 160

TAT CTT TCA ATG TCT GTG GAC ACT GTT ATT CCA CCT GGG GAA    779
Tyr Leu Ser Met Ser Val Asp Thr Val Ile Pro Pro Gly Glu
    165                 170                 175

GAG GCA GTT AAT TCT GAT ATC GCC TGC CTC TAC AAC AGG CCG    821
Glu Ala Val Asn Ser Asp Ile Ala Cys Leu Tyr Asn Arg Pro
    180                 185                 190

ACA ATA CAC CCA TTT GCC TAC AGA GTC CAC ACT CAT CAG TTG    863
Thr Ile His Pro Phe Ala Tyr Arg Val His Thr His Gln Leu
            195                 200                 205

GGG CAG GTC GTA AGT GGA TTT AGA GTG AGA CAT GGC AAG TGG    905
Gly Gln Val Val Ser Gly Phe Arg Val Arg His Gly Lys Trp
            210                 215

TCT TTA ATT GGT AGA CAA AGC CCA CAG CTG CCA CAG GCA TTT    947
Ser Leu Ile Gly Arg Gln Ser Pro Gln Leu Pro Gln Ala Phe
220                 225                 230
```

```
TAC CCT GTA GAG CAT CCA GTA GAG ATT AGC CCT GGG GAT ATT    989
Tyr Pro·Val Glu His Pro Val Glu Ile Ser Pro Gly Asp Ile
    235                 240             245

ATA GCA ACC AGG TGT CTG TTC ACT GGT AAA GGC AGG ACG TCA   1031
Ile Ala Thr Arg Cys Leu Phe Thr Gly Lys Gly Arg Thr Ser
        250                 255             260

GCA ACA TAT ATT GGG GGC ACA TCT AAC GAT GAA ATG TGT AAT   1073
Ala Thr Tyr Ile Gly Gly Thr Ser Asn Asp Glu Met Cys Asn
            265                 270             275

TTA TAC ATC ATG TAT TAC ATG GAT GCG GCC CAT GCT ACG TCA   1115
Leu Tyr Ile Met Tyr Tyr Met Asp Ala Ala His Ala Thr Ser
            280                 285

TAC ATG ACC TGT GTA CAG ACA GGT GAA CCA AAG CTA TTT CAA   1157
Tyr Met Thr Cys Val Gln Thr Gly Glu Pro Lys Leu Phe Gln
290             295                 300

AAC ATC CCT GAG ATT GCA AAT GTT CCC ATT CCT GTA AGC CCT   1199
Asn Ile Pro Glu Ile Ala Asn Val Pro Ile Pro Val Ser Pro
    305                 310                 315

GAC ATG ATG ATG ATG ATG GGA CAT GGT CAC CAC CAT ACA GAA   1241
Asp Met Met Met Met Met Gly His Gly His His His Thr Glu
        320                 325                 330

GCT GAG CCT GAG AAG AAT ACA GGA CTT CAG CAG CCT AAA CGA   1283
Ala Glu Pro Glu Lys Asn Thr Gly Leu Gln Gln Pro Lys Arg
            335                 340                 345

GAG GAG GAA GAA GTA TTA GAT CAG GGT CTC ATT ACC TTA GGG   1325
Glu Glu Glu Glu Val Leu Asp Gln Gly Leu Ile Thr Leu Gly
                350                 355

GAT AGC GCA GTG TGA TGGAGGAGGA CATGATCCCT ATACCGTTGA      1370
Asp Ser Ala Val
360

AGGGGATGAC CCAATCATTT T                                   1391
```

## Claims

1. A process for production of pepridylglycine α-hydroxylating monooxygenase, comprising culturing insect cells transfected with a recombinant baculovirus to which a DNA coding for a peptidylglycine α-hydroxylating monooxygenase has been incorporated to produce the enzyme.

2. A process according to claim 1, wherein a recombinant baculovirus is prepared by inserting a cDNA fragment coding for a peptidylglycine α-hydroxylating monooxygenase into a transfer plasmid comprising a polyhedrin gene to construct a recombinant transfer vector, and inserting said cDNA fragment into a baculovirus genome by homologous recombination.

3. A process according to claim 2, wherein the transfer vector is pAcYM1, and the baculovirus is Autographa californica nuclear polyhedrosis virus (AcMNPV).

4. A process according to claim 1, wherein the peptidylglycine α-hydroxylating monooxygenase is recovered in purified or enriched form.

5. A process for the production of a C-terminal α-hydroxyglycyl peptide comprising converting a C-terminal glycine-extended peptide of the formula (I)

$$R-NH-CH_2-COOH \quad (I)$$

wherein R represents a peptide and -NH-CH$_2$-COOH represents a glycine residue present at the C-terminus of the peptide R, to a C-terminal α-hydroxyglycyl peptide of the formula (II)

$$R-NH-CHOH-COOH \quad (II)$$

using the enzyme prepared by a process according to claim 1.

6. A process according to claim 5, wherein the α-hydroxyglycyl peptide is C-terminally α-hydroxyglycylated human calcitonin, salmon calcitonin, chicken calcitonin, eel calcitonin, rat calcitonin, bovine calcitonin, porcine calcitonin, ovine calcitonin, human growth hormon-releasing factor (GHRF), human calcitonin generatated peptide (CGRP), or luteinizing hormon-releasing hormone (LHRH).

7. A process according to claim 5, wherein the α-hydroxyglycyl peptide is C-terminally α-hydroxyglycylated human calcitonin.

8. A process for the production of a C-terminal α-amidated peptide of the formula R-NH$_2$ (I) comprising converting a C-terminal α-hydroxyglycyl peptide of the formula R-NH-CHOH-COOH (II) by treatment with a base.

9. A process according to claim 8, wherein the starting material of the formula (II) is obtained according to the process of claim 5.

10. A process according to claim 8, wherein the starting material of the formula (II) is C-terminally α-hydroxyglycylated human calcitonin.

11. A calcitonin having an additional n-hydroxyglycine unit on its C-terminus.

12. A calcitonin according to claim 11 which is human calcitonin.

13. A method for assay of peptidylglycine α-hydroxylating monooxygenase, comprising the steps of:
   a) reacting a test sample with a substrate peptide having a glycine residue at the C-terminus in a reaction medium having a pH value optimum for action of the enzyme;
   b) rising the pH value of the reaction mixture to at least pH 10;
   c) neutralize the medium to a neutral pH; and
   d) determining an amount of formed C-terminal amidated peptide.

## Claims for the following Contracting States: ES, GR

1. A process for production of peptidylglycine α-hydroxylating monooxygenase, comprising culturing insect cells transfected with a recombinant baculovirus to which a DNA coding for a peptidylglycine α-hydroxylating monooxygenase has been incorporated to produce the enzyme.

2. A process according to claim 1, wherein a recombinant baculovirus is prepared by inserting a cDNA fragment coding for a peptidylglycine α-hydroxylating monooxygenase into a transfer plasmid comprising a polyhedrin gene to construct a recombinant transfer vector, and inserting said cDNA fragment into a baculovirus genome by homologous recombination.

3. A process according to claim 2, wherein the transfer vector is pAcYM1, and the baculovirus is Autographa californica nuclear polyhedrosis virus (AcMNPV).

4. A process according to claim 1, wherein the peptidylglycine α-hydroxylating monooxygenase is recovered in purified or enriched form.

5. A process for the production of a C-terminal α-hydroxyglycyl peptide comprising converting a C-terminal glycine-extended peptide of the formula (I)

$$R\text{-}NH\text{-}CH_2\text{-}COOH \quad (I)$$

wherein R represents a peptide and -NH-CH$_2$-COOH represents a glycine residue present at the C-terminus of the peptide R, to a C-terminal α-hydroxyglycyl peptide of the formula (II)

$$R\text{-}NH\text{-}CHOH\text{-}COOH \quad (II)$$

using the enzyme prepared by a process according to claim 1.

6. A process according to claim 5, wherein the α-hydroxyglycyl peptide is C-terminally α-hydroxyglycylated human calcitonin, salmon calcitonin, chicken calcitonin, eel calcitonin, rat calcitonin, bovine calcitonin, porcine calcitonin, ovine calcitonin, human growth hormon-releasing factor (GHRF), human calcitonin generatated peptide (CGRP), or luteinizing hormon-releasing hormone (LHRH).

7. A process according to claim 5, wherein the α-hydroxyglycyl peptide is C-terminally α-hydroxyblycylated human calcitonin.

8. A process for the production of a C-terminal α-amidated peptide of the formula R-NH$_2$ (I) comprising converting a C-terminal α-hydroxyglycyl peptide of the formula R-NH-CHOH-COOH (II) by treatment with a base.

9. A process according to claim 8, wherein the starting material of the formula (II) is obtained according to the process of claim 5.

10. A process according to claim 8, wherein the starting material of the formula (II) is C-terminally α-hydroxyglycylated human calcitonin.

11. A method for assay of peptidylglycine α-hydroxylating monooxygenase, comprising the steps of:
    a) reacting a test sample with a substrate peptide having a glycine residue at the C-terminus in a reaction medium having a pH value optimum for action of the enzyme;
    b) rising the pH value of the reaction mixture to at least pH 10;
    c) neutralize the medium to a neutral pH;
    and
    d) determining an amount of formed C-terminal amidated peptide.

OKAYAMA—BERG
CLONING VECTOR

*Fig. 1—1*

pAE—I—310

pSVL

Sma I          Dra I

Sma I    BamH I

DIGEST WITH
RESTRICTION ENZYMES
Sma I AND Dra I

DIGEST WITH
RESTRICTION ENZYMES
Sma·I,
DEPHOSPHORYLATION WITH
ALKALINE PHOSPHATASE

LIGATE

pSVL/AE—I—310

Bam HI

Pst I

14

# Fig. 1 – 2

BACULOVIRUS TRANSFER VECTOR

pSVL/AE I–310

cDNA of PAM

Pst I          Bam HI

pAcYM1

BamHI

DIGEST WITH RESTRICTION ENZYMES
Pst I AND BamHI, FILL IN WITH T4
DNA POLYMERASE,
LIGATE TO Bam HI LINKERS,
DIGEST WITH RESTRICTION
ENZYME BamHI

DIGEST WITH RESTRICTION
ENZYME BamHI
DEPHOSPHORYLATE WITH
ALKALINE PHOSPHATASE

LIGATE

RECOMBINANT BV TRANSFER VECTOR

pAcYM1/AE–I

POLYHEDRIN
PROMOTOR

AUG    Term.

BamHI        PAM
cDNA

# Fig. 2

pAcYM1/AE-I

AUTOGRAPHA CALOFORNICA
NUCLEAR POLYHEDROSIS VIRUS

WILD-TYPE AcNPV DNA

Co-TRANSFECTION

INSECT CELLS

4-5 DAYS INCUBATION

HARVEST SUPERNATANT

PLAQUE ASSAY

HOMOLOGOUS RECOMBINATION

POLYHEDRIN-NEGATIVE
CLEAR-PLAQUE

WILD-TYPE
WHITE PLAQUE  CLEAR-PLAQUE ISOLATION

AMPLIFICATION

RECOMBINANT VIRUS DNA

POLYHEDRIN
PROMOTOR

# Fig. 3

# Fig. 4